# EUROPÄISCHE PATENTANMELDUNG

(11) **EP 2 615 359 A1**
(43) Veröffentlichungstag der Anmeldung: **17.07.2013**
(21) Anmeldenummer: 13151424.2
(22) Anmeldetag: 16.01.2013
(51) Int. Cl.: F21K 99/00, A61B 19/00

(54) **Optische Diagnosevorrichtung**

(30) Priorität: 16.01.2012 DE 102012000562
(71) Anmelder: Ludwig Leuchten KG, 86415 Mering (DE)
(72) Erfinder: Wichnalek, Dr. Lydia, 86420 Kreppen (DE); Wichnalek, Dr. Willi, 86420 Kreppen (DE); Weisser, Wolfgang, 73457 Essingen (DE); Ludwig, Alexander, 86492 Heinrichshofen (DE)
(74) Vertreter: Meissner, Bolte & Partner

(57) **Zusammenfassung**

Die Erfindung betrifft eine optische Diagnosevorrichtung (10, 20, 30), welche eine Beleuchtungsvorrichtung (12, 22, 32) zum Beleuchten eines zu behandelnden Objekts sowie eine Tragevorrichtung (14, 24, 34) zum Tragen der Beleuchtungsvorrichtung (12, 22, 32) aufweist. Um den Farbton eines zu behandelnden Objekts möglichst objektiv beurteilen zu können ist es bei der erfindungsgemäßen optischen Diagnosevorrichtung (10, 20, 30) vorgesehen, dass die Beleuchtungsvorrichtung (12, 22, 32) mindestens ein Beleuchtungselement, mit einem Farbwiedergabeindex welcher größer als 90 ist, aufweist und dazu ausgelegt ist, das zu behandelnde Objekt mit einer Farbtemperatur von 5000 K bis 6000 K, vorzugsweise bis 5500 K zu beleuchten.

## Beschreibung

Die Erfindung betrifft eine optische Diagnosevorrichtung, insbesondere für zahnärztliche Verwendung, gemäß dem Oberbegriff des unabhängigen Patentanspruchs 1.

Demgemäß weist die erfindungsgemäße optische Diagnosevorrichtung eine Beleuchtungsvorrichtung zum Beleuchten eines zu behandelnden Objekts sowie eine Tragevorrichtung zum Tragen der Beleuchtungsvorrichtung auf.

In der Medizin ist es seit langer Zeit gängige Praxis, ein zu behandelndes Objekt mit einer medizinischen Beleuchtungsvorrichtung mit geeigneter Beleuchtungsstärke zu beleuchten. Insbesondere ist es aus der Dentalmedizin bekannt, medizinische Beleuchtungsvorrichtungen am vorderen Ende eines Hängearms anzubringen um damit den Mundraum eines Patienten für die dentale Behandlung hell zu beleuchten. Eine herkömmliche medizinische Beleuchtungsvorrichtung verfügt über eine solche Konfiguration, bei der eine Halogenlampe als Lichtquelle verwendet wird und ein zu behandelndes Objekt mit durch eine Spiegelplatte reflektierendem Licht beleuchtet wird.

Gerade in der Zahnmedizin bzw. im Dentallabor hat neben der Zahnstellung, der Zahnform und der Oberflächenstruktur der Zähne vor allem die Zahnfarbe eine entscheidende Bedeutung. Insbesondere für die Bestimmung eines geeigneten Zahnersatzes ist es ausschlaggebend, wie gut der Zahnersatz mit den im Mund befindlichen näheren Nachbarzähnen harmonisiert. Es ist aus diesem Grund bekannt, die Zahnfarbe durch metrische Verfahren zu bestimmen. Dabei registrieren Farbmessgeräte das vom zu Behandelnde Objekt (Zahn) zurückgestrahlte Lichtspektrum (Remissionssprektrum), um reproduzierbare Farbwerte zu berechnen, die einen Vergleich von Farben erlauben. Zwar sind die derart erzielten Messergebnisse in hohem Masse reproduzierbar, jedoch hat sich gezeigt, dass diese nicht immer mit dem visuellen Farbeindruck eines menschlichen Betrachters übereinstimmen. Darüber hinaus haben die erwähnten Farbmessgeräte einen hohen Anschaffungspreis und sind somit nicht weiträumig einsetzbar.

Eine weitere aus dem Stand der Technik bekannte Methode ist die farbliche Klassifizierung natürlicher Zahnfarben mit Hilfe eines Zahnmusterschlüssels. Dabei ist der Behandelnde bei der Bestimmung der Zahnfarbe auf die Farbwahrnehmungsfähigkeiten seiner Augen angewiesen. Die Farbwahrnehmung des menschlichen Auges hängt jedoch von einer Vielzahl von äußeren Faktoren, wie bspw. Beleuchtung, Hintergrundfarben, Kleidung und Ermühung des Auges, ab.

Gerade die aus dem Stand der Technik bekannten Beleuchtungsvorrichtungen sind oftmals nicht ideal zur objektiven Farbbestimmung der natürlichen Zähne, da diese keine idealen Farbcharakteristiken aufweisen. Ferner sind die herkömmlichen Halogen-Behandlungsleuchten zur Auswahl der Farben nicht geeignet, da sie mit etwa 8000 lux die Zähne weitaus zu hell erscheinen lassen.

Auf Grund oben genannter Problemstellung ist es die Aufgabe der vorliegenden Erfindung, eine optische Diagnosevorrichtung anzugeben, welche dazu dient den Farbton eines zu behandelnden Objekts möglichst objektiv beurteilen zu können.

Zur Lösung der oben stehenden Aufgabe wird eine erfindungsgemäße optische Diagnosevorrichtung gemäß dem gekennzeichneten Teil des unabhängigen Patentanspruchs 1 vorgeschlagen.

Demgemäß betrifft die Erfindung eine optische Diagnosevorrichtung, bei der die Beleuchtungsvorrichtung mindestens ein Beleuchtungselement, mit einem Farbwiedergabeindex welcher größer als 90 ist, aufweist und dazu ausgelegt ist, das zu behandelnde Objekt mit einer Farbtemperatur von 5000 K bis 6000 K, vorzugsweise bis 5500 K zu beleuchten.

Die erfindungsgemäße optische Diagnosevorrichtung weist eine Vielzahl von Vorteilen auf. So wird durch den Farbwiedergabeindex von größer 90 eine optimale Farbauflösung durch das menschliche Auge erreicht, wodurch eine fehlerfreie Beurteilung des Farbtons der natürlichen Zähne durch den Betrachter begünstigt wird. Eine Farbtemperatur von 5000 K bis 6000 K entspricht in etwa der spektralen Verteilung von Tageslicht und hat den Vorteil, dass keine Verfälschung des Farbeindrucks entsteht. So käme es bei einer Farbtemperatur unter 5000 K zu einer Rotverschiebung, während Farbetemperaturen über 6000 K zu einer Blauverschiebung führen würden. Insbesondere ist es vorteilhaft eine Farbtemperatur von 5250 bis 5500 K zu verwenden, da sich herausgestellt hat, dass hierdurch der beste Farbeindruck gewonnen werden kann. Dieser Farbeindruck ist jedoch nur dann aussagekräftig wenn der Farbwiedergabeindex des verwendeten Beleuchtungselements über 90 liegt. Ferner sei an dieser Stelle erwähnt, dass die Beleuchtungsvorrichtung der erfindungsgemäßen optischen Diagnosevorrichtung vorzugsweise eine Beleuchtungsstärke von 2000 lux aufweist um somit das zu behandelnde Objekt ausreichend hell, jedoch nicht zu hell erscheinen zu lassen.

Weitere Ausführungsformen der erfindungsgemäßen optischen Diagnosevorrichtung sind in den Unteransprüchen angegeben.

So ist es in einer ersten Weiterbildung der erfindungsgemäßen optischen Diagnosevorrichtung vorgesehen, dass die Beleuchtungsvorrichtung zum Ausbilden einer Apertur ringförmig ausgebildet ist und eine Vielzahl von Beleuchtungselementen aufweist, welche auf einer Kreislinie angeordnet sind. Die Ausbildung der Beleuchtungsvorrichtung in einer Ringform, hat den Vorteil, dass in der Mitte des Ringes eine Apertur entsteht, durch welche der Betrachter ideale Sichtverhältnisse auf das zu behandelnden Objekt hat. Wie später näher erläutert werden wird, kann die durch die Ringform gebildete Apertur ferner dazu genutzt werden ein Bildaufnahmegerät mit der erfindungsgemäßen optischen Diagnosevorrichtung zu verbinden. Im Zusammenhang mit dieser Ausführungsform sei insbesondere erwähnt, dass es von Vorteil ist, eine Vielzahl von Beleuchtungselementen auf einer Kreislinie anzuordnen, um eine möglichst homogene Ausleuchtung des kompletten Behandlungsbereichs zu erzielen.

Das mindestens eine Beleuchtungselement der erfindungsgemäßen optischen Diagnosevorrichtung ist vorzugsweise als organische oder anorganische Leuchtdiode ausgebildet. Im Gegensatz zu den herkömmlichen als Lichtquellen verwendeten Halogenlampen bzw. Leuchtstoffröhren weisen die Beleuchtungselemente, welche als Leuchtdioden ausgebildet sind eine sehr viel höhere Lebensdauer auf und besitzen insbesondere eine Farbtemperatur, welche auch über einen längeren Zeitraum konstant bleibt. Leuchtdioden eignen sich insbesondere deshalb als Beleuchtungselemente für die erfindungsgemäße optische Diagnosevorrichtung, da diese ohnehin ausgelegt ist, im Nahbereich des zu behandelnden Objekts verwendet zu werden.

Insbesondere ist es bevorzugt etwa 9 Leuchtdioden äquidistant auf einer einzigen Kreislinie um die Apertur herum anzuordnen. Es sei in diesem Zusammenhang ferner erwähnt, dass Leuchtdioden bei hohen Farbtemperaturen, wie den erfindungsgemäßen 5000 K bis 6000 K oftmals einen relativ geringen Farbwiedergabeindex (kleiner 90) aufweisen. Dennoch hat sich überraschenderweise herausgestellt, dass es möglich ist die gewünschten Farbeigenschaften der erfindungsgemäßen optischen Diagnosevorrichtung auch durch den Einsatz von Leuchtdioden zu erzielen.

Gemäß einer weiteren erfindungsgemäßen Ausgestaltung der optischen Diagnosevorrichtung weist die Beleuchtungsvorrichtung eine Abbildungseinrichtung auf, welche zwischen dem zu behandelnden Objekt und dem mindestens einen Beleuchtungselement angeordnet ist. Bei der Abbildungseinrichtung kann es sich vorzugsweise um optische Linsen bzw. Spiegelelemente handeln, welche in der Beleuchtungsvorrichtung integriert sind. Auch ist es denkbar, dass die eben erwähnte Abbildungseinrichtung einen oder mehrere Farbfilter zur Optimierung der Farbtemperatur aufweist. Durch die Abbildungsseinrichtung wird gewährleistet, dass der gesamte Bereich der zu behandelnden Objekte optimal beleuchtet werden kann und die Beeinträchtigung des Patienten auf ein minimales Maß reduziert wird.

Nach einer weiteren Ausführungsform weist die Tragevorrichtung eine Tragstruktur mit einem horizontal ausgerichteten oberen Endbereich auf, wobei der obere Endbereich dazu ausgebildet ist ein Bildaufnahmegerät zu tragen. Somit kann die optische Diagnosevorrichtung ohne Weiteres mit einem beliebigen Bildaufnahmegerät verbunden werden, wodurch sich reproduzierbare Aufnahmen des zu behandelnden Objekts erstellten lassen.

Die Tragstruktur kann dabei mindestens ein erstes Befestigungselement an dem zuvor genannten oberen Endbereich aufweisen, welches dazu ausgelegt ist, ein Bildaufnahmegerät lösbar mit der Tragstruktur zu verbinden. Dementsprechend lassen sich verschiedene Bildaufnahmegeräte schnell und einfach mit der optischen Diagnosevorrichtung verbinden, wobei diese zugleich sicher an der Tragstruktur der Diagnosevorrichtung angebracht sind.

Weiterhin ist es von Vorteil, wenn der obere Endbereich der Tragstruktur mindestens eine erste Führungsnut aufweist, um ein Bildaufnahmegerät in einer ersten, im Wesentlichen horizontalen Richtung gegenüber der Beleuchtungsvorrichtung auszurichten. Durch die mindestens eine erste Führungsnut lässt sich die Linse eines Bildaufnahmegeräts besonders einfach gegenüber dem zu behandelnden Objekt ausrichten, wodurch eine optimale Fokussierung mit sehr geringem Aufwand erzielt werden kann.

Gemäß einem weiteren Aspekt der erfindungsgemäßen optischen Diagnosevorrichtung ist es vorgesehen, dass die Tragevorrichtung ein Positionierelement aufweist, welches lösbar mit der Beleuchtungsvorrichtung verbunden ist und dazu ausgelegt ist, die Beleuchtungsvorrichtung in einer im Wesentlichen vertikalen Richtung gegenüber einem Bildaufnahmegerät auszurichten. Demgemäß lässt sich bspw. die Position der Apertur der Beleuchtungsvorrichtung sehr schnell auf die Position der Eingangslinse des Bildaufnahmegeräts ausrichten. Dies sorgt für eine optimale Ausbeute der von der Beleuchtungsvorrichtung bereitgestellten Lichtmenge.

Gleichzeitig kann das Positionierelement dazu ausgelegt sein, die Beleuchtungsvorrichtung in einer zweiten, im Wesentlichen horizontalen Richtung gegenüber einem Bildaufnahmegerät auszurichten, wobei die zweite horizontale Richtung im Wesentlichen senkrecht zur ersten horizontalen Richtung verläuft. Auf diese Weise kann die erfindungsgemäße optische Diagnosevorrichtung in allen drei Raumrichtungen verstellt werden, wodurch sich der Fokus eines Bildaufnahmegeräts optimal auf das zu behandelnde Objekt einstellen lässt.

Alternativ hierzu kann die optische Diagnosevorrichtung ein Adapterelement, mit mindestens einer zweiten Führungsnut aufweisen. Gemäß dieser Ausführungsvariante ist nicht das Positionierelement, sondern die zweite Führungsnut dazu ausgebildet, ein Bildaufnahmegerät in einer zweiten, im Wesentlichen horizontalen Richtung gegenüber der Beleuchtungsvorrichtung auszurichten, wobei die zweite horizontale Richtung im Wesentlichen senkrecht zu der ersten horizontalen Richtung verläuft. Wie eben bereits erwähnt, lässt sich auch durch das Adapterelement eine besonders einfache Ausrichtung verschiedener Bildaufnahmegeräte in alle drei Raumrichtungen erzielen.

Das Adapterelement kann dabei ein zweites Befestigungselement zum lösbaren Befestigen eines Bildaufnahmegeräts an dem Adapterelement aufweisen. Folglich kann ein Bildaufnahmegerät einfach und schnell an dem Adapterelement befestigt werden, während dieses wiederum zuverlässig an der ersten Führungsnut der Tragestruktur angebracht werden kann. Nach einer weiteren Umsetzung der optischen Diagnosevorrichtung kann die Beleuchtungsvorrichtung ein Sensorelement zum Ein/Ausschalten sowie Dimmen der Beleuchtungsvorrichtung aufweisen. Vorzugsweise kann es sich dabei um ein Tastelement handeln, welches sich auf der Rückseite der Beleuchtungsvorrichtung befindet. Dementsprechend kann die Beleuchtungsstärke der Beleuchtungsvorrichtung stufenlos vom Benutzer eingestellt werden.

Die optische Diagnosevorrichtung kann darüber hinaus eine Steuervorrichtung aufweisen, welche dazu ausgelegt ist, die Lichtintensität sowie die Lichtverteilung der Beleuchtungsvorrichtung zu steuern. Insbesondere kann diese Steuervorrichtung ein Programm zur Auswahl einer oder mehrere Beleuchtungselemente der Beleuchtungsvorrichtung aufweisen. Somit ist es vorstellbar, nur einzelne Beleuchtungselemente der Beleuchtungsvorrichtung anzusteuern, während andere Beleuchtungselemente ausgeschaltet bleiben, um die Lichtverteilung auf einen Bereich des zu behandelnden Objekts zu richten, welcher von besonderem Interesse ist.

Nach einer weiteren Ausführungsform weist die Tragevorrichtung der optischen Diagnosevorrichtung eine Tragstruktur auf, mit welcher mindestens ein in einer ersten Richtung verschiebbares Positionierelement, zum Positionieren der Beleuchtungsvorrichtung gegenüber dem zu behandelnden Objekt, verbunden ist. Durch das in einer ersten Richtung bewegbare Positionierelement ist es auf einfache Weise möglich, den Fokus der Beleuchtungsvorrichtung optimal auf den Bereich der zu behandelnden Objekte einzustellen. Durch die einfache Einstellbarkeit des Lichtfokuses ist gewährleistet, dass jederzeit eine besonders günstige Beleuchtungsintensität erreicht wird.

Es ist besonders vorteilhaft, das verschiebbare Positionierelement mit einem Flanschbereich auszustatten, welcher zum lösbaren Befestigen der Beleuchtungsvorrichtung dient. Auf diese Weise ist es möglich, die Beleuchtungsvorrichtung der erfindungsgemäßen optischen Diagnosevorrichtung jederzeit schnell und einfach auszuwechseln. Dazu kann das verschiebbare Positionierelement bspw. über ein Befestigungselement mit der Beleuchtungsvorrichtung verbunden sein. Es ist insbesondere denkbar, den Flanschbereich derart auszuführen, dass die Beleuchtungsvorrichtung im gewissen Rahmen gegenüber dem verschiebbaren Positionierelement verstellt werden kann.

Einer weiteren Realisierung der optischen Diagnosevorrichtung zu Folge weist die Tragstruktur einen im Wesentlichen T-förmigen Querschnitt mit einem oberen Endbereich zum Aufnehmen eines Bildaufnahmegeräts auf. Durch die Ausbildung der Tragstruktur mit einem im Wesentlichen T-förmigen Querschnitt kann ein oberer Endbereich geschaffen werden, welcher als Ablage für ein herkömmliches Bildaufnahmegerät dient. Zur Ausbildung der Tragstruktur mit einem im Wesentlichen T-förmigen Querschnitt, kann diese bspw. als Alu-Standsbiegeteil gefertigt werden. Durch die Möglichkeit zur Aufnahme eines Bildaufnahmegeräts, wie bspw. einer Spiegelreflexkamera, Kompaktkamera oder gar eines Smartphones, ist es durch die erfindungsgemäße optische Diagnosevorrichtung nicht nur möglich das zu behandelnde Objekt subjektiv durch den Beobachter zu beurteilen, sondern eine optimale Abbildung dessen zu erzeugen. In diesem Zusammenhang könnte die erfindungsgemäße optische Diagnosevorrichtung auch dazu genutzt werden, eine Farbveränderung der zu behandelnden Objekte, insbesondere Zähne, über einen längeren Zeitraum zu speichern und zu vergleichen und zwar unter reproduzierbaren Bedingungen (identische Beleuchtung der Zähne, identischer Abstand zur Kamera, usw.)

Die eben erwähnte Tragstruktur kann in vorteilhafter Weise an ihrem oberen Endbereich ein erstes Befestigungselement aufweisen, welches dazu ausgelegt ist, dass Bildaufnahmegerät lösbar mit der Tragstruktur zu verbinden. Durch die Anordnung des Befestigungselements am oberen Endbereich, ist es möglich ein Bildaufnahmegerät optimal und sicher in Bezug auf die Beleuchtungsvorrichtung zu positionieren, um somit eine bestmögliche Bildqualität zu gewährleisten.

Gemäß einer weiteren Ausführungsform der erfindungsgemäßen optischen Diagnosevorrichtung weist diese ein Bildaufnahmegerät auf, welches über das erste Befestigungselement lösbar mit der Tragstruktur verbunden ist. Wie oben bereits angedeutet hat die Verwendung eines Bildaufnahmegeräts den Vorteil, dass digitale Abbildungen der zu Behandelnden Objekte einfach und sicher archiviert werden können. Dies kann beispielsweise die Herstellung von ästhetischem Zahnersatz durch einen Zahntechniker begünstigen, welcher sich hierdurch ein sehr genaues Bild des gewünschten Endprodukts (Farbe, Kontur, usw.) machen kann. Durch den Einsatz von Bildaufnahmegeräten, wie bspw. Spiegelreflexkameras, können Feinstrukturen des zu behandelnden Objekts sichtbar gemacht werden, welche für das menschliche Auge nicht ohne Weiteres erkennbar wären. Auch können die gewonnenen digitalen Abbildungen durch bekannte Bildbearbeitungs-Programme auf die individuellen Bedürfnisse des Behandlers/Technikers angepasst werden.

Es sei an dieser Stelle angemerkt, dass die erfindungsgemäße optische Diagnosevorrichtung nicht nur dazu dienen kann ein Bildaufnahmegerät zu tragen. Vielmehr ist die erfindungsgemäße optische Diagnosevorrichtung dazu ausgelegt, eine perfekte, schattenfreie Ausleuchtung des zu behandelnden Objekts bereitzustellen, um mit einem Referenzmuster (z.B. Zahnfarbring, keramisch gebranntem Materialmuster, Composite, usw.) ein farbverbindliches Arbeitsbild/Referenzbild zu erstellen. Wie oben bereits erwähnt, wird dies insbesondere durch die Beleuchtung des zu behandelnden Objekts mit einer Farbtemperatur von 5000 K bis 6000 K, bei einem Farbwiedergabeindex von größer 90, erreicht.

Darüber hinaus kann die optische Diagnosevorrichtung gemäß einer weiteren Umsetzung eine Führungsvorrichtung am oberen Endbereich der Tragstruktur aufweisen, welche derart ausgelegt ist, sodass das mindestens eine erste Befestigungselement in einer zweiten Richtung bewegbar ist, wobei die zweite Richtung senkrecht zur ersten Richtung ist. Die mit einer Führungsvorrichtung ausgestattete optische Diagnosevorrichtung ist somit sehr leicht auf eine Vielzahl unterschiedlicher Bildaufnahmengeräte anpassbar.

Gemäß einer weiteren Ausführungsform weist die Tragstruktur eine verschiebbare Kabelführung, zum Führen elektrischer Vorsorgungsleitungen für das mindestens eine Beleuchtungselement, auf. Die verschiebbare Kabelführung dient insbesondere dazu, dass die elektrischen Versorgungsleitungen für das mindestens eine Beleuchtungselement angemessen geführt werden, selbst wenn die Beleuchtungsvorrichtung durch das Positionierelement gegenüber der Tragstruktur bewegt wird. Somit wird effektiv verhindert, dass die elektrischen Versorgungsleitungen das Wohlbefinden des Patienten während der Behandlung beeinträchtigen. Anzumerken ist, dass es sein kann die erfindungsgemäße optische Diagnosevorrichtung mit Akkus oder Batterien zu betreiben. Dies ist insbesondere bei der Verwendung von Leuchtdioden als Beleuchtungselemente vorstellbar. Dementsprechend könnte auf eine Kabelführung auch gänzlich verzichtet werden.

Im Folgenden wird die erfindungsgemäße optische Abführungsvorrichtung mit Bezug auf das in den Figuren dargestellte Ausführungsbeispiel näher beschrieben.

Es zeigen:
- Fig. 1:: Perspektivische Vorderansicht einer ersten Ausführungsform der erfindungsgemäßen optischen Diagnosevorrichtung;
- Fig. 2:: perspektivische Rückansicht der in Fig. 1 dargestellten ersten Ausführungsform der erfindungsgemäßen optischen Diagnosevorrichtung;
- Fig. 3a:: Vorderansicht einer zweiten Ausführungsform der erfindunsgemäßen optischen Diagnosevorrichtung;
- Fig. 3b:: Seitenansicht der in Fig. 3a dargestellten zweiten Ausführungsform;
- Fig. 3c:: Draufsicht auf die in Fig. 3a dargestellte zweite Ausführungsform;
- Fig. 4:: perspektivische Ansicht der in Fig. 3a dargestellten zweiten Ausführungsform;
- Fig. 5a:: Vorderansicht einer dritten Ausführungsform der erfindungsgemäßen optischen Diagnosevorrichtung;
- Fig. 5b:: Seitenansicht der in Fig. 5a dargestellten dritten Ausführungsform;
- Fig. 5c:: Draufsicht auf die in Fig. 5a dargestellte dritte Ausführungsform;
- Fig. 5d:: perspektivische Ansicht eines Adapterelements;
- Fig. 6:: perspektivische Ansicht der in Fig. 3a dargestellten dritten Ausführungsform;
- Fig. 7:: perspektivische Rückansicht der in Fig. 3a dargestellten zweiten Ausführungsform mit alternativen Bildaufnahmegeräten.

In der folgenden detaillierten Figurenbeschreibung sind gleiche oder gleich wirkende Bauteile mit gleichen Bezugszeichen versehen. Insbesondere sind die Bezugszeichen vergleichbarer Bauteile der ersten, zweiten und dritten Ausführungsform jeweils um den Faktor "10" bzw. "100" oder "1000" erhöht bzw. erniedrigt.

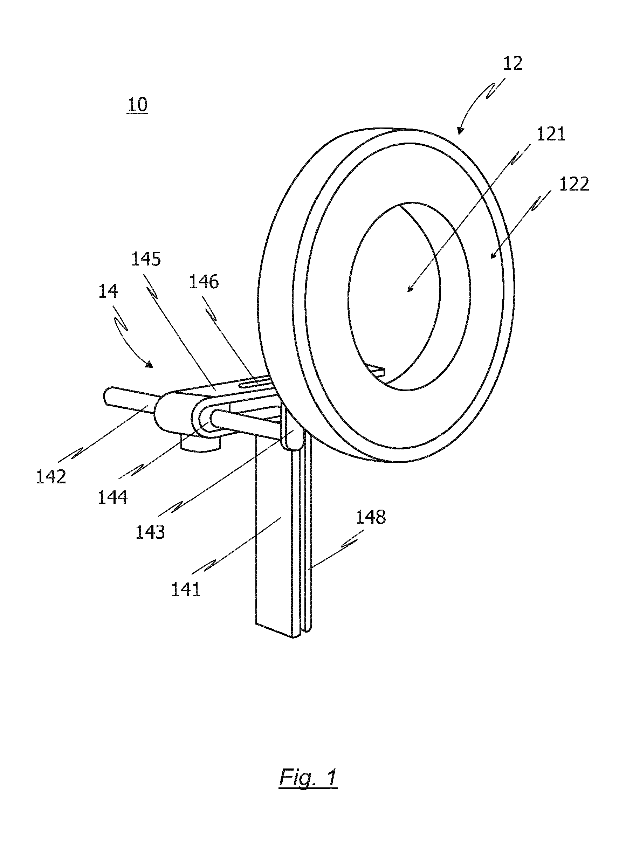

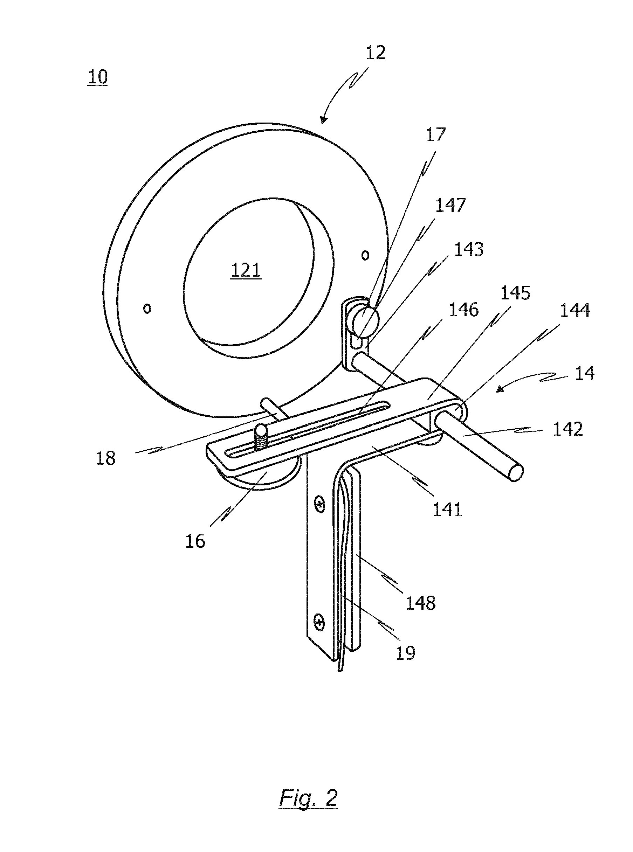

Eine erste Ausführungsform der erfindungsgemäßen optischen Diagnosevorrichtung 10 ist in Fig. 1 und 2 dargestellt. Im Einzelnen weist die erfindungsgemäße optische Diagnosevorrichtung 10 eine Beleuchtungsvorrichtung 12 zum Beleuchten eines zu behandelnden Objekts (nicht dargestellt) sowie eine Tragevorrichtung 14 zum Tragen der Beleuchtungsvorrichtung 12. Die Beleuchtungsvorrichtung 12 weist mindestens ein Beleuchtungselement (nicht dargestellt), mit einem Farbwiedergabeindex welcher größer als 90 ist, auf und ist dazu ausgelegt, das zu behandelnde Objekt mit einer Farbtemperatur von 5000 K bis 6000 K, vorzugsweise 5000 K bis 5500 K zu beleuchten.

Wie es den Darstellungen zu entnehmen ist, ist es insbesondere von Vorteil, die erfindungsgemäße Beleuchtungsvorrichtung 12 ringförmig auszubilden, wodurch eine Apertur 121 im Mittelpunkt der ringförmigen Beleuchtungsvorrichtung 12 entsteht. Es ist in diesem Zusammenhang denkbar, dass eine Vielzahl von Beleuchtungselementen auf einer Kreislinie innerhalb der Beleuchtungsvorrichtung 12 angeordnet sind. Mit anderen Worten sind die Beleuchtungselemente rund um die Apertur 121 angeordnet. Somit kann die Beleuchtungsvorrichtung 12 dazu genutzt werden, das zu behandelnde Objekt möglichst homogen im kompletten Behandlungsbereich auszuleuchten. Dabei ist es vorgesehen, dass ein Großteil des von den Beleuchtungselementen emittierten Lichts von dem zu behandelnden Objekt reflektiert wird und durch die Apertur 121 tritt.

Um die erfindungsgemäße optische Diagnosevorrichtung besonders langlebig und energiesparend auszugestalten, ist es vorgesehen die Beleuchtungselemente als Leuchtdioden auszubilden. Diese können sehr einfach hinter einer Abbildungseinrichtung 122 innerhalb der Beleuchtungsvorrichtung 12 angebracht werden. Dementsprechend ist es vorgesehen, dass die Beleuchtungsvorrichtung 12 eine Abbildungseinrichtung 122 aufweist, welche zwischen dem zu behandelnden Objekt und dem mindestens einen Beleuchtungselement angeordnet ist. Bei der in den Fig. 1 und 2 dargestellten Abbildungseinrichtung 122 handelt es sich vorzugsweise um eine optische Linse, welche am Gehäuse der Beleuchtungsvorrichtung 12 befestigt ist. Selbstverständlich ist es auch denkbar, dass die Beleuchtungsvorrichtung darüber hinaus Spiegelelemente im Inneren aufweist, welche dazu dienen das von den Beleuchtungselementen emittierte Licht in Richtung des zu behandelnden Objekts zu reflektieren. Es sei darauf hingewiesen, dass die Beleuchtungselemente vorzugsweise derart innerhalb der Beleuchtungsvorrichtung angeordnet sein sollten, sodass die Beeinträchtigung (Blendung) des Patienten auf ein minimales Maß reduziert wird.

Vor allem der Darstellung gemäß Fig. 2 ist zu entnehmen, dass die Tragevorrichtung 14 der erfindungsgemäßen optischen Diagnosevorrichtung 10 eine Tragstruktur 141 aufweist, mit welcher mindestens ein in einer ersten Richtung bewegbares Positionierelement 142, zum Positionieren der Beleuchtungsvorrichtung 12 gegenüber dem zu behandelnden Objekt, verbunden ist. Dabei kann es insbesondere vorgesehen sein, dass die Tragstruktur 141 als Alu-Standbiegeteil ausgebildet ist. Im Einzelnen kann das Alu-Standbiegeteil durch Kaltumformung in den dargestellten T-förmigen Zustand gebracht werden. Die dargestellte Tragstruktur umfasst ein Führungselement 144, welches bspw. als eingefrästes Kunststoffteil ausgebildet sein kann. In der dargestellten Ausführungsform weist das Führungselement 144 eine Durchgangsbohrung auf, in welcher das in einer ersten Richtung bewegbare Positionierelements 142 geführt wird. Es sei an dieser Stelle erwähnt, dass das Führungselement 144 selbstverständlich auch mittig and er Tragstruktur 141 (nicht dargestellt) angebracht sein kann. Das Positionierelement 142 kann demnach insbesondere dazu genutzt werden, den Lichtfokus der Beleuchtungsvorrichtung 12 genau auf das zu behandelnde Objekt (z.B. Zähne des Patienten) einzustellen. Somit wird eine schattenfreie Beleuchtung des zu behandelnden Objekts durch die erfindungsgemäße optische Diagnosevorrichtung gewährleistet.

Das bewegbare Positionierelement 142 ist als Stiftelement ausgebildet, welches an seinem vorderen Ende einen Flanschbereich 143, zum lösbaren Befestigen der Beleuchtungsvorrichtung 12, aufweist. Im Einzelnen ist es vorgesehen, dass das bewegbare Positionierelement 142 über den Flanschbereich, mit Hilfe eines zweiten Befestigungselements 17 lösbar mit der Beleuchtungsvorrichtung 12 verbunden werden kann. Um eine Feinjustierung der Beleuchtungsvorrichtung 12 gegenüber der Tragevorrichtung 14 zu gewährleisten, kann ein Langloch 147 im Flanschbereich 143 vorgesehen sein. Somit kann die Beleuchtungsvorrichtung 12 zumindest Bereichsweise vertikal gegenüber der Tragevorrichtung 14 verstellt werden. Zwar ist das bewegbare Positionierelement 142 in den Darstellungen seitlich an der Rückseite der Beleuchtungsvorrichtung 12 angebracht, jedoch ist es alternativ natürlich auch denkbar das Positionierelement 142 an allen anderen Positionen (z.B. mittig) an der Beleuchtungsvorrichtung 12 anzubringen.

Wie oben bereits angedeutet, weist die Tragstruktur 141 einen im Wesentlichen T-förmigen Querschnitt mit einem oberen Endbereich 145 zum Aufnehmen eines Bildaufnahmegeräts auf. Mit anderen Worten wird durch den T-förmigen Querschnitt der Tragstruktur 141 eine gerade Oberfläche (oberer Endbereich 145) geschaffen, welche dazu benutzt werden kann ein Bildaufnahmegerät auf der letzteren zu positionieren. Dementsprechend ist die Tragevorrichtung 14 der erfindungsgemäßen optischen Diagnosevorrichtung 10 dazu in der Lage jegliches Bildaufnahmegerät, wie bspw. eine Spiegelreflexkamera, Kompaktkamera oder Smartphones aufzunehmen. Zu diesem Zweck kann es jedoch nötig sein, eine spezielle Adapterhalterung (z.B. für Smartphones) vorzusehen, welche mit einem ersten Befestigungselement 16 an der Tragstruktur 141 befestigt ist. Alternativ kann das Bildaufnahmegerät natürlich auch direkt über das erste Befestigungselement 16 an der Tragstruktur 141 befestigt werden.

Wie oben bereits erwähnt können durch die spezielle Anordnung der erfindungsgemäßen optischen Diagnosevorrichtung 10 besonders genau reproduzierbare Beleuchtungsbedingungen bereitgestellt werden. Somit wird gewährleistet, dass auch über einen längeren Zeitraum digitale Abbildungen des zu behandelnden Objekts, unter identischen Bedingungen (Beleuchtung, Kamerafokus, usw.), erzeugt werden können.

Das erste Befestigungselement 16 kann in einer Führungsnut 146 am oberen Endbereich 145 der Tragstruktur 141 bewegbar sein. Zu diesem Zweck ist die Führungsnut 146 der Tragstruktur 141 derart ausgebildet, sodass das erste Befestigungselement in einer zweiten Richtung bewegbar ist, welche senkrecht zur ersten Richtung verläuft. Die Führungsnut 146 kann, wie dargestellt, als Langloch ausgebildet sein, wobei es jedoch selbstverständlich auch denkbar ist andere Mittel zur Führung, wie bspw. Schwalbenschwanznuten, vorzusehen.

In Fig. 2 ist ferner dargestellt, dass die Tragstruktur 141 eine verschiebbare Kabelführung 18 zum Führen elektrischer Vorsorgungsleitungen 19 für das mindestens eine Beleuchtungselement 12 aufweist. Die verschiebbare Kabelvorrichtung 18 umfasst die elektrische Vorsorgungsleitung 19 und ist wie auch das Positionierelement 142 in einer ersten Richtung bewegbar. Ein weiterer Kabelkanal wird durch ein Seitenteil 148 ausgebildet, welches über Befestigungselemente (z.B. Schrauben) mit der Tragstruktur 141 verbunden ist. Im Einzelnen kann das Seitenteil 148 dabei als gefräster Kunststoffgriff ausgebildet sein, welcher eine Kehle zur Führung der elektrischen Vorsorgungsleitungen 19 aufweist.

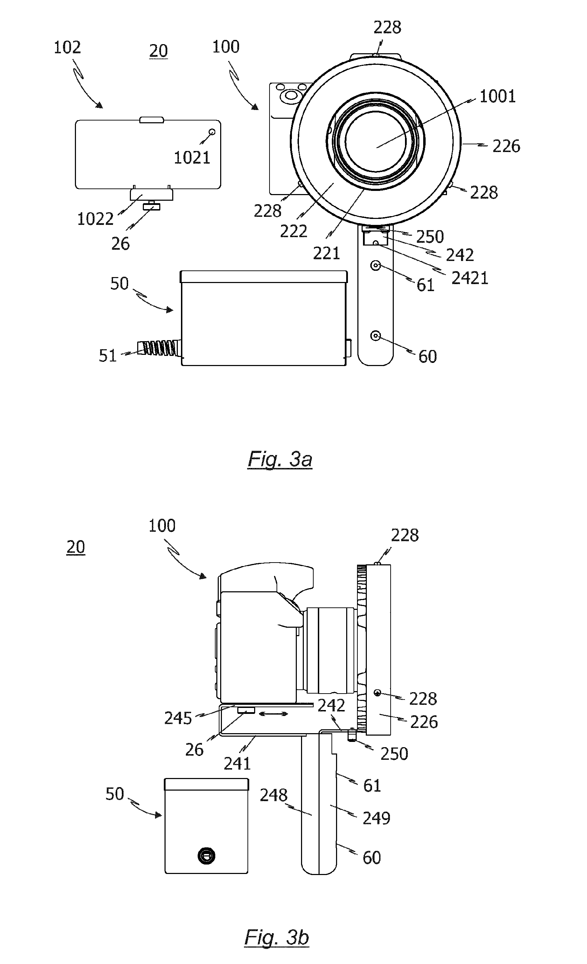

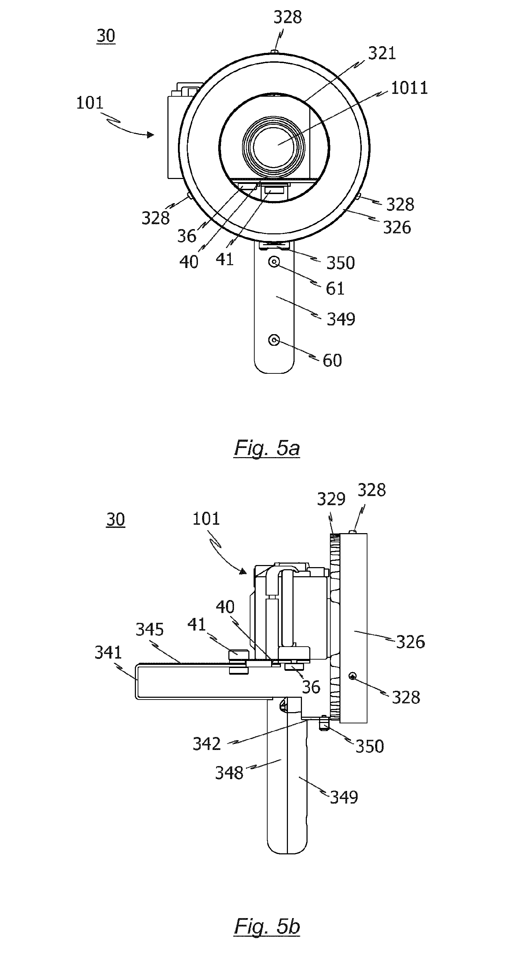

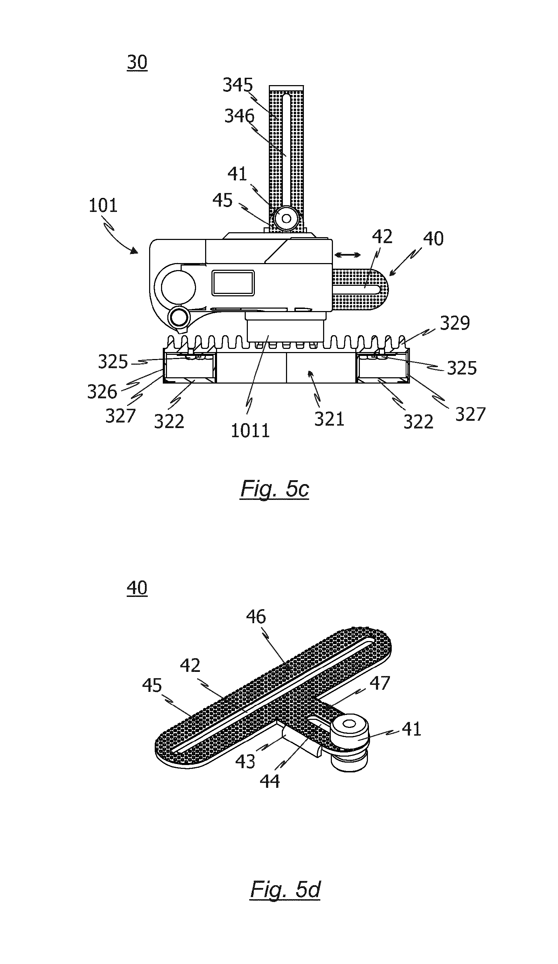

Eine zweite Ausführungsform der erfindungsgemäßen optischen Diagnosevorrichtung ist den Fig. 3a bis 4 zu entnehmen. Wie es sich aus den Darstellungen der Fig. 3a bis 3c erkennen lässt, weist auch die Tragvorrichtung 24 der zweiten Ausführungsform eine Tragstruktur 241 mit einem horizontal ausgerichteten oberen Endbereich 245 auf. Der obere Endbereich 245 der Tragstruktur 241 ist insbesondere dazu ausgebildet, ein Bildaufnahmegerät 100 aufzunehmen. Wie es später näher erläutert werden wird, kann es sich bei dem Bildaufnahmegerät um verschiedene Vorrichtungen wie bspw. Spiegelreflexkameras 100, Kompaktkameras 101 (Fig. 5) oder Smartphones 102 handeln.

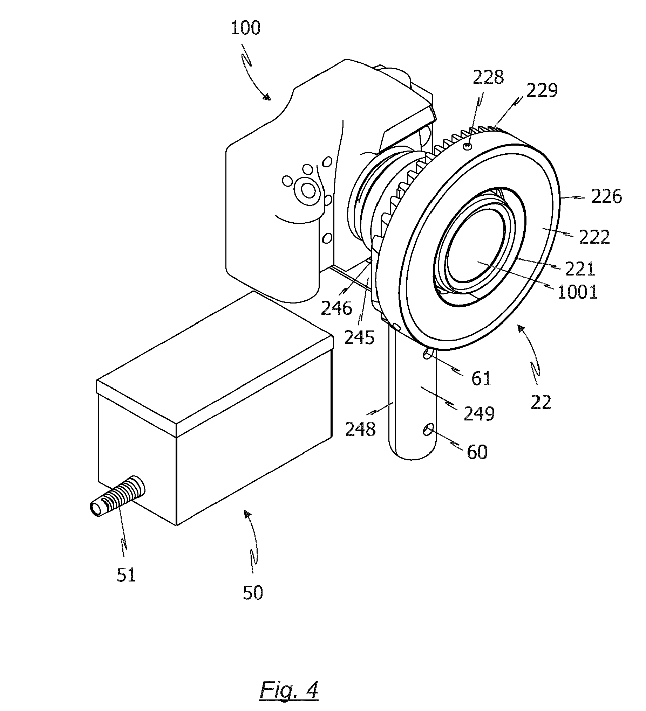

Auch die Tragstruktur 241 der optischen Diagnosevorrichtung 20 gemäß der zweiten Ausführungsform weist ein erstes Befestigungselement 26 an dem oberen Endbereich 245 auf, welches dazu ausgelegt ist, ein Bildaufnahmegerät 100, 101, 102 lösbar mit der Tragstruktur 241 zu verbinden. Aus den Darstellungen gemäß Fig. 3b und Fig. 4 ist ersichtlich, dass der obere Endbereich 245 der Tragstruktur 241 mindestens eine erste Führungsnut 246 zum Ausrichten eines Bildaufnahmegeräts (hier Spiegelreflexkamera 100) gegenüber der Beleuchtungsvorrichtung 22 in einer ersten, im Wesentlichen horizontalen Richtung, aufweist. Im Einzelnen ermöglicht die Führungsnut 246, dass das Bildaufnahmegerät 100, 101, 102 auf die Beleuchtungsvorrichtung 22 zu bzw. von der Beleuchtungsvorrichtung weg bewegt werden kann. Dies ist beispielsweise durch den in Fig. 3b dargestellten Doppelpfeil, am Beispiel einer Spiegelreflexkamera 100 angedeutet.

In den Darstellungen gemäß den Fig. 3a und 3b ist gezeigt, dass die optische Diagnosevorrichtung gemäß der zweiten Ausführungsform weiterhin ein Positionierelement 242 aufweist, welches mit der Beleuchtungsvorrichtung 24 verbunden ist und dazu ausgelegt ist, die Beleuchtungsvorrichtung 24 in einer im Wesentlichen vertikalen Richtung gegenüber einem Bildaufnahmegerät 100 auszurichten.

Im Einzelnen ist das Positionierelement 242 dabei als Schienenelement ausgebildet, welches in den Darstellungen gemäß Fig. 3a und 3b nach oben bzw. nach unten verschoben werden kann. Dies wird insbesondere durch einen unteren Teilbereich der Tragvorrichtung 24 ermöglicht. Dieser untere Teilbereich weist einen hinteren Abschnitt 248 sowie einen vorderen Abschnitt 249 auf, wobei der vordere Abschnitt 249 über Befestigungselemente 60, 61 mit dem hinteren Abschnitt 248 in Verbindung steht. Wie es durch die Fig. 3a angedeutet ist, weist das Positionierelement 242 eine Vielzahl von Durchgangsöffnungen 2421 auf, welche sich entlang des zwischen dem vorderen Abschnitt 249 und dem hinteren Abschnitt 248 befindlichen Teilbereichs des Positionierelements 242 befinden. Mit anderen Worten ist das Positionierelement 242 verschiebbar zwischen dem vorderen Abschnitt 249 und dem hinteren Abschnitt 248 gelagert, wobei sich die Befestigungselemente 60, 61 durch jeweilige Durchgangsöffnungen 2421 des Positionierelements 242 erstrecken. Somit wird das Positionierelement 242 in der gewünschten vertikalen Position befestigt. Durch ein Lösen der Befestigungselemente 60 und 61 kann das Positionierelement 242 und somit die Beleuchtungsvorrichtung 22 in vertikaler Richtung verschoben werden. Eine derartige Verstellung ist bspw. zum Anpassen der optischen Diagnosevorrichtung auf das jeweilige Bildaufnahmegerät 100, 101, 102 notwendig, wie es aus einem Vergleich der zweiten und dritten Ausführungsform ersichtlich ist (Fig. 3a und Fig. 5a).

Alternativ oder zusätzlich hierzu kann es selbstverständlich auch vorgesehen sein, die Tragstruktur 241, 341 vertikal verschiebbar auszugestalten. Um die Höhenverstellbarkeit zu vereinfachen, kann die oben erwähnte Verstelleinrichtung (Positionierelement und Befestigungselemente) auch ohne die Befestigungselemente 60, 61 ausgebildet sein. Beispielsweise kann es hierzu vorgesehen sein, dass ein Druckschalter (nicht dargestellt) an der Tragevorrichtung 24, 34 angebracht ist, bei dessen Betätigung ein Verschieben des Positionierelements 242, 342 bzw. der Tragstruktur 241, 341 in vertikaler Richtung ermöglicht wird. Der Druckschalter kann mit einer schwingenden exzentrischen Lasche versehen sein, welche das Positionierelement 242, 342 bzw. die Tragstruktur 241, 341, im nicht betätigten Zustand, fixiert und bei Betätigung eine vertikale Verschiebung freigibt.

Weiterhin ist zu erkennen, dass die optische Diagnosevorrichtung 20 auch eine Steuervorrichtung 50 aufweisen kann, welche dazu ausgelegt ist, die Lichtintensität sowie die Lichtverteilung der Beleuchtungsvorrichtung 24 zu steuern. Zu diesem Zweck ist die Steuervorrichtung 50 über einen Anschlussstutzen 51 und einer nicht dargestellten Verbindungsleitung mit den innerhalb der Beleuchtungsvorrichtung 22 angebrachten Beleuchtungselementen 223 verbunden. Wie in den Fig. 3a und 3b angedeutet, kann die Haltevorrichtung 24 zu diesem Zweck einen lastabtragenden Kabelauslass 250 aufweisen, welcher dazu ausgebildet ist, die Verbindungsleitung sofort nachdem Verlassen der Kühlrippen 224 abzustützen. Die Steuervorrichtung kann gleichzeitig als Energieversorgung (Akku) für die Beleuchtungselemente 223 der Beleuchtungsvorrichtung 22 dienen.

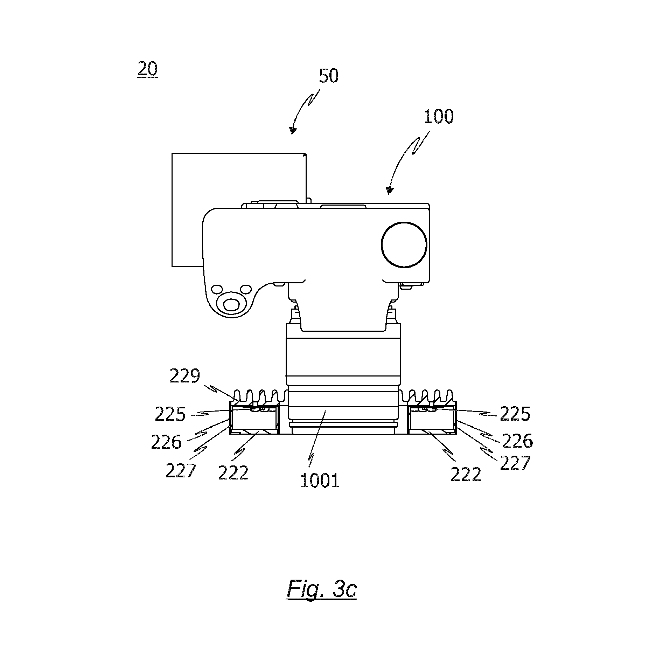

Aus der Fig. 3c ist eine Draufsicht der in Fig. 3a dargestellten Ausführungsform der erfindungsgemäßen Diagnosevorrichtung 20 zu erkennen. Dabei ist die Beleuchtungsvorrichtung 22 im Schnitt dargestellt. Wie es sich hieraus erkennen lässt, sind auch hier die Beleuchtungselemente 225 auf einer Kreislinie innerhalb der Beleuchtungsvorrichtung 22 angeordnet. Die Beleuchtungsvorrichtung 22 selbst weist ein ringförmiges Gehäuse 226 auf, welches an seinen Innenwänden mit einer reflektierenden Beschichtung 227 versehen ist. Am vorderen Endbereich des Gehäuses 226 befindet sich eine Abbildungseinrichtung 222, welche dazu dient, das von den Beleuchtungselementen produzierte Licht optimal auf das zu behandelnde Objekt zu fokussieren. Wie oben bereits erwähnt, sollte das Objektiv 1001 des Bildaufnahmegeräts 100 vorzugsweise zentral innerhalb der ringförmigen Beleuchtungsvorrichtung 22 angeordnet sein. Es sei an dieser Stelle angemerkt, dass die Beleuchtungsvorrichtung 12 gemäß der ersten Ausführungsform vorzugsweise einen identischen Aufbau wie die Beleuchtungsvorrichtung 22 der zweiten Ausführungsform aufweist.

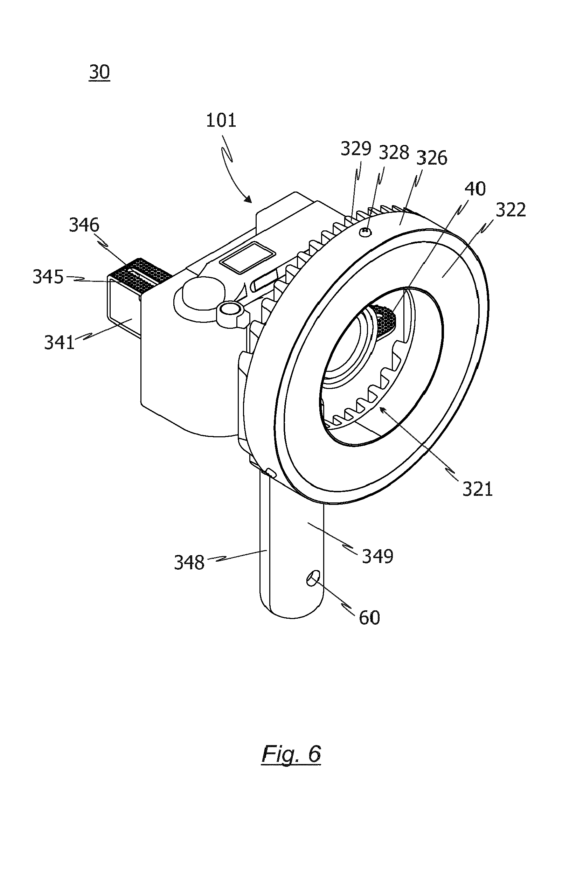

Eine dritte Ausführungsform der erfindungsgemäßen optischen Diagnosevorrichtung ist den Fig. 5a bis 6 zu entnehmen. Im Gegensatz zu der zweiten Ausführungsform gemäß der Fig. 3a bis 4, ist bei der dritten Ausführungsform eine Kompaktkamera 101 anstelle der Spiegelreflexkamera 100 vorgesehen. Um auch diese Kompaktkamera 101 optimal bezüglich der Apparatur 321 der Beleuchtungsvorrichtung 32 ausrichten zu können, ist gemäß er dritten Ausführungsform ferner ein Adapterelement 40 mit mindestens einer zweiten Führung 46 vorgesehen, welches dazu ausgebildet ist, ein Bildaufnahmegerät (hier Kompaktkamera 101) in einer zweiten, im Wesentlichen horizontalen Richtungen gegenüber der Beleuchtungsvorrichtung 32 auszurichten. Dabei ist die zweite, horizontale Richtung im Wesentlichen senkrecht zur ersten, horizontalen Richtung des ersten Führungselements 346 (Fig. 5c) der Tragstruktur 341.

Der Darstellung gemäß Fig. 5d ist weiterhin zu entnehmen, dass das Adapterelement 40 ein zweites Befestigungselement 41 zum lösbaren Befestigen des Adapterelements 40 an der ersten Führungsnut 346 aufweist. Somit kann das Adapterelement 40 auf einfache Weise an dem oberen Endbereich 345 der Haltevorrichtung 34 befestigt werden und ist gleichzeitig, wie bereits die Spiegelreflexkamera 100 aus der zweiten Ausführungsform, in einer im Wesentlichen ersten horizontalen Richtung verschiebbar. Das Bildaufnahmegerät 101 bzw. 102 ist dabei über ein erstes Befestigungselement 36 an der zweiten Führungsnut 42 des Adapterelements 40 befestigt. Wie bereits erwähnt, kann das Bildaufnahmegerät 101 bzw. 102 dadurch in einer zweiten, durch den Doppelpfeil in Fig. 5c angedeuteten horizontalen Richtung gegenüber der Beleuchtungsvorrichtung 32 bewegt werden.

Das Adapterelement 40 weist einen vorderen Teilbereich 46 auf, an welchem die zweite Führungsnut 42 angebracht ist. An einem hinteren Teilbereich 47 befindet sich eine weitere, dritte Führungsnut 44, welche sich entlang der ersten, im Wesentlichen horizontalen Richtung erstreckt. Somit erstreckt sich die dritte Führungsnut 44 parallel zur ersten Führungsnut 346 des oberen Endbereichs 345, wie dies aus Figur 5c ersichtlich ist. Mit Hilfe der dritten Führungsnut 44, lässt sich das Adapeterelement individuell auf den Platzbedarf der verschiedenen Bildaufnahmegeräte, wie Kompaktkameras 101 oder Smartphones 102, einstellen.

Um eine gerade Führung des Adapterelements gegenüber dem oberen Endbereich 345 der Tragstruktur 341 zu gewährleisten, sind Führungsschienen 43 an den Seitenbereichen des Adapterelements 40 vorgesehen. Diese werden vor der Montage des Befestigungselements 41 auf den oberen Endbereich 345 der Tragstruktur 341 aufgeschoben.

Schließlich sei angemerkt, dass der obere Endbereich 145, 245, 345 der jeweiligen Tragstrukturen 141, 241, 341 jeweils mit einer Anti-Rutsch Beschichtung versehen sein kann, um ein Bildaufnahmegerät sicher auf dem oberen Endbereich platzieren zu können. Alternativ dazu oder gleichzeitig kann das Adapterelement 40 gemäß der dritten Ausführungsform ebenfalls eine derartige Anti-Rutsch Beschichtung 45 aufweisen, wie dies durch die Fig. 5c und 5d angedeutet ist.

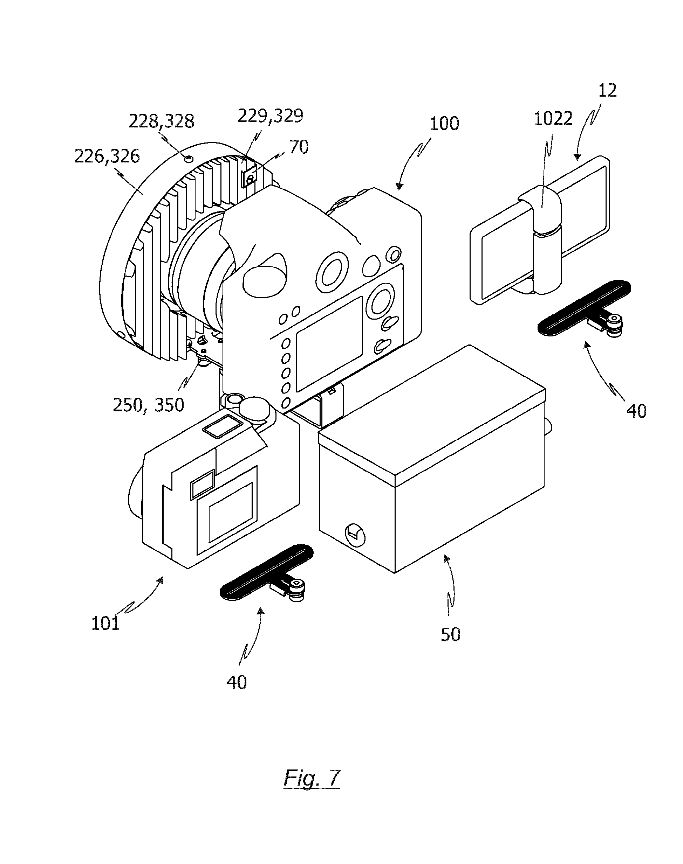

Mit Bezug auf die perspektivische Rückansicht gemäß Fig. 7 sei erwähnt, dass die Beleuchtungsvorrichtung 12, 22, 32 vorzugsweise ein Sensorelement 70 zum Ein/Ausschalten sowie Dimmen der Beleuchtungsvorrichtung 12, 22, 32 aufweisen kann. Dieses Sensorelement 70 ist vorzugsweise auf der Rückseite der Beleuchtungsvorrichtung 12, 22 bzw. 32 vorgesehen. Es ist jedoch selbstverständlich auch möglich, das Sensorelement an jeder beliebigen Stelle der Diagnosevorrichtung anzubringen. Beispielsweise könnte dieses auch am vorderen bzw. hinteren Abschnitt 248, 249, 348, 349 der Tragevorrichtung 24, 34 vorgesehen sein, um die Bedienung komfortabler zu gestalten. Dies ist insbesondere dann vorteilhaft, wenn die Tragevorrichtung 24 bzw. 34 als Handgriff ausgebildet ist. Somit kann die Beleuchtungsvorrichtung durch das Tastfeld des Sensorelements 70 jederzeit während der Behandlung mit nur einer Hand ein-/ausgeschalten bzw. gedimmt werden.

Es lässt sich ferner aus der Fig. 7 erkennen, dass die optische Diagnosevorrichtung gemäß der zweiten Ausführungsform sehr einfach auf verschiedene Bildaufnahmegeräte 100, 101 bzw. 102 angepasst werden kann. Dazu ist es lediglich notwendig, das beschriebene Adapterelement 40 (Fig. 5d) mit der Tragstruktur 241 zu verbinden und somit gemäß der dritten Ausführungsform das als Kompaktkamera oder Smartphone 101 bzw. 102 ausgebildete Bildaufnahmegerät gegenüber der Beleuchtungsvorrichtung 22 bzw. 32 auszurichten. Es sei an dieser Stelle ferner angemerkt, dass es im Falle eines Smartphones ferner notwendig ist, eine Haltevorrichtung 1022 vorzusehen, über die das als Smartphone ausgebildete Bildaufnahmegerät 102 mit dem Adapterelement 40 verbunden werden kann.

### Bezugszeichenliste

- 10, 20, 30: optische Diagnosevorrichtung
- 12, 22, 32: Beleuchtungsvorrichtung
- 14, 24, 34: Tragevorrichtung
- 16, 26, 36: erstes Befestigungselement
- 17: zweites Befestigungselement
- 18: Kabelführung
- 19: elektrische Vorsorgungsleitung
- 40: Adapterelement
- 41: zweites Befestigungselement
- 42: zweite Führungsnut
- 43: Führungsschiene
- 44: dritte Führungsnut
- 45: Anti-Rutsch-Beschichtung
- 46: vorderer Teilbereich
- 47: hinterer Teilbereich
- 50: Steuervorrichtung
- 51: Anschlussstutzen
- 60, 61: Befestigungselement
- 100, 101, 102: Bildaufnahmegerät
- 121, 221, 321: Apertur
- 122, 222, 322: Abbildungseinrichtung
- 141, 241, 341: Tragstruktur
- 142, 242: Positionierelement
- 143: Flanschbereich
- 144: Führungselement
- 145, 245, 345: oberer Endbereich
- 146, 246, 346: erste Führungsnut
- 147: Langloch
- 148: Seitenteil
- 225, 325: Beleuchtungselement
- 226, 326: Gehäuse
- 227, 327: reflektierende Beschichtung
- 228, 328: Befestigungsschrauben
- 229, 329: Kühlrippen
- 248, 348: vorderer Abschnitt
- 249, 349: hinterer Abschnitt
- 250, 350: Kabelauslass
- 1001, 1011, 1021: Linse
- 1022: Haltevorrichtung
- 2421: Durchgangsöffnung

## Patentansprüche

1. Optische Diagnosevorrichtung (10, 20, 30) für die zahnärztliche Verwendung, welche eine Beleuchtungsvorrichtung (12, 22, 32) zum Beleuchten eines zu untersuchenden Objekts sowie eine Tragevorrichtung (14, 24, 34) zum Tragen der Beleuchtungsvorrichtung (12, 22, 32) aufweist,
**dadurch gekennzeichnet, dass**
die Beleuchtungsvorrichtung (12, 22, 32) mindestens ein Beleuchtungselement mit einem Farbwiedergabeindex größer als 90 aufweist und dazu ausgelegt ist, das zu behandelnde Objekt mit einer Farbtemperatur von 5000 K bis 6000 K zu beleuchten.

2. Optische Diagnosevorrichtung (10, 20, 30) nach Anspruch 1,
wobei die Beleuchtungsvorrichtung (12, 22, 32) zum Ausbilden einer Apertur (121, 221, 321) ringförming ausgebildet ist und eine Vielzahl von Beleuchtungselementen aufweist, welche auf einer Kreislinie angeordnet sind.

3. Optische Diagnosevorrichtung (10, 20, 30) nach Anspruch 1 oder 2,
wobei das mindestens eine Beleuchtungselement als Leuchtdiode ausgebildet ist.

4. Optische Diagnosevorrichtung (10, 20, 30) nach einem der vorhergehenden Ansprüche,
wobei die Beleuchtungsvorrichtung (12, 22, 32) eine Abbildungseinrichtung (122, 222, 322) aufweist, welche zwischen dem zu behandelnden Objekt und dem mindestens einen Beleuchtungselement angeordnet ist.

5. Optische Diagnosevorrichtung (10, 20, 30) nach einem der vorhergehenden Ansprüche,
wobei die Tragevorrichtung (14, 24, 34) eine Tragstruktur (141, 241, 341) mit einem horizontal ausgerichteten oberen Endbereich (145, 245, 345) aufweist, wobei der obere Endbereich (145, 245, 345) dazu ausgebildet ist ein Bildaufnahmegerät (100, 101, 102) zu tragen.

6. Optische Diagnosevorrichtung nach Anspruch 5,
wobei die Tragstruktur (141, 241, 341) mindestens ein erstes Befestigungselement (16, 26, 36) an dem oberen Endbereich (145, 245, 345) aufweist, welches dazu ausgelegt ist, ein Bildaufnahmegerät (100, 101, 102) lösbar mit der Tragstruktur (141, 241, 341) zu verbinden.

7. Optische Diagnosevorrichtung (10, 20, 30) nach Anspruch 6,
wobei der obere Endbereich (145, 245, 345) der Tragstruktur (141, 241, 341) mindestens eine erste Führungsnut (146, 246, 346), zum Ausrichten eines Bildaufnahmegeräts (100, 101, 102) gegenüber der Beleuchtungsvorrichtung (12, 22, 32) in einer ersten, im Wesentlichen horizontalen Richtung, aufweist.

8. Optische Diagnosevorrichtung (10, 20, 30) nach einem der Ansprüche 5 bis 7,
wobei die Tragevorrichtung ein Positionierelement (142, 242, 342) aufweist, welches lösbar mit der Beleuchtungsvorrichtung (12, 22, 32) verbunden ist und dazu ausgelegt ist, die Beleuchtungsvorrichtung (12, 22, 32) in einer im Wesentlichen vertikalen Richtung gegenüber einem Bildaufnahmegerät (100, 101, 102) auszurichten.

9. Optische Diagnosevorrichtung (10) nach Anspruch 8,
wobei das Positionierelement (142) ferner dazu ausgelegt ist, die Beleuchtungsvorrichtung (12) in einer zweiten, im Wesentlichen horizontalen Richtung gegenüber einem Bildaufnahmegerät (100, 101, 102) auszurichten, wobei die zweite horizontale Richtung im Wesentlichen senkrecht zur ersten horizontalen Richtung verläuft.

10. Optische Diagnosevorrichtung (30) nach einem der Ansprüche 1 bis 8, wobei die optische Diagnosevorrichtung ein Adapterelement (40), mit mindestens einer zweiten Führungsnut (42) aufweist, welche dazu ausgebildet ist, ein Bildaufnahmegerät (100, 101, 102) in einer zweiten, im Wesentlichen horizontalen Richtung gegenüber der Beleuchtungsvorrichtung (32) auszurichten, wobei die zweite horizontale Richtung im Wesentlichen senkrecht zur ersten horizontalen Richtung verläuft.

11. Optische Diagnosevorrichtung (30) nach Anspruch 10 in Kombination mit einem der Ansprüche 7 oder 8,
wobei das Adapterelement (40) ein zweites Befestigungselement (41) zum lösbaren befestigen des Adapterelements (40) an der ersten Führungsnut (346) aufweist.

12. Optische Diagnosevorrichtung (10, 20, 30) nach einem der vorhergehenden Ansprüche,
wobei die Beleuchtungsvorrichtung (12, 22, 32) ein Sensorelement (70) zum Ein-/Ausschalten sowie Dimmen der Beleuchtungsvorrichtung (12, 22, 32) aufweist.

13. Optische Diagnosevorrichtung (10, 20, 30) nach einem der vorhergehenden Ansprüche,
wobei die optische Diagnosevorrichtung ein Bildaufnahmegerät (100, 101, 102) aufweist, welches lösbar mit der Tragstruktur (141, 241, 341) verbunden ist.

14. Optische Diagnosevorrichtung (10, 20, 30) nach einem der vorhergehenden Ansprüche,
wobei die Optische Diagnosevorrichtung (10, 20, 30) eine Steuervorrichtung (50) aufweist, welche dazu ausgelegt ist, die Lichtintensität sowie die Lichtverteilung der Beleuchtungsvorrichtung (12, 22, 32) zu Steuern.
